# EUROPEAN PATENT SPECIFICATION

(11) **EP 4 340 728 B1**
(45) Date of publication and mention of the grant of the patent: **01.07.2026**
(21) Application number: 22727631.8
(22) Date of filing: 12.05.2022
(51) Int. Cl.: A61B 6/00, A61B 6/04, A61B 6/50

(54) **SYSTEMS AND METHODS FOR MACHINE LEARNING BASED OPTIMAL EXPOSURE TECHNIQUE PREDICTION FOR ACQUIRING MAMMOGRAPHIC IMAGES**
SYSTEME UND VERFAHREN ZUR OPTIMALEN BELICHTUNGSTECHNIKVORHERSAGE AUF DER BASIS VON MASCHINELLEM LERNEN ZUR ERFASSUNG VON MAMMOGRAPHIEBILDERN
SYSTÈMES ET MÉTHODES DE PRÉDICTION DE TECHNIQUE D'EXPOSITION OPTIMALE BASÉE SUR L'APPRENTISSAGE AUTOMATIQUE POUR L'ACQUISITION D'IMAGES MAMMOGRAPHIQUES

(30) Priority: 18.05.2021 US 202163189953 P
(43) Date of publication of application: 27.03.2024
(73) Proprietor: Hologic, Inc., Marlborough, MA 01752 (US)
(72) Inventor: KSHIRSAGAR, Ashwini, Marlborough, MA 01752 (US); REN, Baorui, Marlborough, MA 01752 (US); SMITH, Andrew, P., Marlborough, MA 01752 (US)
(74) Representative: Carpmaels & Ransford LLP
(86) International application number: PCT/US2022/028901
(87) International publication number: WO 2022/245619

(56) References cited:
- WO-A1-2020/216307
- US-A1- 2008 103 834
- US-A1- 2011 257 919
- US-A1- 2015 199 478
- US-A1- 2020 381 125
- US-A1- 2022 133 258

## Description

### BACKGROUND

Achieving optimal automatic exposure control (AEC) is essential for generating a mammographic image having adequate contrast-to-noise ratio without excessive radiation exposure to the imaging subject (e.g., patient). Current AEC techniques for mammography involve sampling the patient's breast to identify the densest regions. In many cases, the sampling does not accurately identity the densest breast regions. As a result, the AEC technique calculated for the identified region may cause a patient to experience excessive radiation exposure ("overexposure") or inadequate radiation exposure ("underexposure").

It is with respect to these and other general considerations that the aspects disclosed herein have been made. Also, although relatively specific problems may be discussed, it should be understood that the examples should not be limited to solving the specific problems identified in the background or elsewhere in the present disclosure.

US2015199478A1 and US2020381124A1 disclose conventional systems and methods for optimizing the exposure parameters for imaging.

### SUMMARY

The invention is set out in the appended set of claims.

Examples of the present disclosure describe systems and methods for using machine learning (ML) to predict optimal exposure technique for acquiring mammographic images. In aspects, a dataset of training data associated with patient data and/or imaging data may be collected from one or more data sources. The dataset of training data may comprise sample data of patient attribute data for a set of patients and sample image attribute data, image metadata, image pixel data, and/or exposure technique parameters for a set of images. The dataset of training data may be used to train an ML model to determine the optimal exposure technique parameters for acquiring mammographic images of a patient. After the ML model has been trained, a dataset of non-training patient data that is collected from a patient during a patient visit may be provided as input to the ML model. Using the patient data, the ML model may output optimal exposure technique parameters for the patient in real-time. The real-time output of the ML model may then be used to generate one or more mammographic images for the patient.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Additional aspects, features, and/or advantages of examples will be set forth in part in the description which follows and, in part, will be apparent from the description, or may be learned by practice of the disclosure.

### BRIEF DESCRIPTION OF THE DRAWINGS

Non-limiting and non-exhaustive examples are described with reference to the following figures.
Figure 1 illustrates an overview of an example system for using ML to predict optimal exposure technique for acquiring mammographic images, as described herein.
Figure 2 is a diagram of an example process flow for using ML to predict optimal exposure technique for acquiring, as described herein.
Figure 3 illustrates an example method for training an ML model for predicting optimal exposure technique for acquiring mammographic images, as described herein
Figure 4 illustrates an example method for using ML to predict optimal exposure technique for acquiring mammographic images, as described herein.
Figure 5 illustrates one example of a suitable operating environment in which one or more of the present embodiments may be implemented.

### DETAILED DESCRIPTION

Medical imaging has become a widely used tool for identifying and diagnosing abnormalities, such as cancers or other conditions, within the human body. Medical imaging processes such as mammography and tomosynthesis are particularly useful tools for imaging breasts to screen for, or diagnose, cancer or other lesions within the breasts. Tomosynthesis systems are mammography systems that allow high resolution breast imaging based on limited angle tomosynthesis. Tomosynthesis, generally, produces a plurality of X-ray images, each of discrete layers or slices of the breast, through the entire thickness thereof. In contrast to conventional two-dimensional (2D) mammography systems, a tomosynthesis system acquires a series of X-ray projection images, each projection image obtained at a different angular displacement as the X-ray source moves along a path, such as a circular arc, over the breast. In contrast to conventional computed tomography (CT), tomosynthesis is typically based on projection images obtained at limited angular displacements of the X-ray source around the breast. Tomosynthesis reduces or eliminates the problems caused by tissue overlap and structure noise present in 2D mammography imaging.

Automatic exposure control (AEC) is a feature that is incorporated into mammographic and radiographic imaging systems. The AEC method functions to enable consistently optimal image exposure despite variations in tissue density and thickness, and user skill level. Achieving optimal AEC is essential for preventing patient overexposure and generating mammographic images that provide a consistent optical density/signal-to-noise ratio. The traditional method for performing AEC in mammography relies on sampling a patient's breast and searching for the densest regions of the breast. While this technique is mostly reliable, the performance of this technique is based on factors such as positioning of the patient, patient-specific anomalies (e.g., the presence of implants or other foreign objects), and consistency in breast compression hardware and procedures. For example, a patient may be improperly positioned such that the AEC search area includes an extremely thick region of the patient's breast (such as the pectoralis muscle). The AEC exposure technique may be calculated for the thick region. As a result, the patient may receive an amount of radiation exposure that is consistent with the thick region, which needlessly overexposes the patient. As another example, a patient may be improperly positioned such that the AEC search area does not include the densest region of the patient's breast. The AEC exposure technique may be calculated for the less dense regions of the breast. As a result, the patient may be underexposed, which may result in poor (or unusable) image quality.

Additionally, the traditional method for performing AEC in mammography requires that a scout image of a patient's breast be taken. The scout image is an initial exposure that is used to identify the amount of radiation exposure needed for the mammographic exam. The AEC search region of the scout image may encompass the entire scout image or may be a subset of the entirety of the scout image, and it may vary, for example, depending upon the breast orientation during the exposure. In many cases, the scout image may be approximately 5-10% of the total amount of radiation exposure and may not even be used in the generation of the final image.

To address such issues with traditional methods for performing AEC, the present disclosure describes systems and methods for using machine learning (ML) to predict optimal exposure technique for acquiring mammographic images. In aspects, training data (or sample data) may be collected from one or more data sources, such as an image repository and/or patient records. The collection of the training data may be performed manually or using an automated process. The training data may comprise patient data for a set of patients (e.g., breast thickness, breast density, breast size, implant/foreign object identifiers), imaging data for a set of images (e.g., pixel data, imaging mode, paddle type) an/or exposure technique parameters for a set of images (e.g., kilovoltage peak (kVp), milliamp-seconds (mAs), X-ray filter combination). The training data may be unlabeled and/or labeled using, for example, text indicators, numerical values, highlighting, encircling (and/or other types of content enclosing), arrows or pointers, font or style modifications, or other forms of annotation.

The training data may be used to train an artificial intelligence (AI) model or an ML model. For example, the training data may be provided as one or more input parameters to an ML model or an ML modelling component. A model, as used herein, may refer to a predictive or statistical utility or program that may be used to determine a probability distribution over one or more character sequences, classes, objects, result sets or events, and/or to predict a response value from one or more predictors. A model may be based on, or incorporate, one or more rule sets, AI/ML, a neural network, or the like. After a model has been trained, the trained model may be able to determine the optimal exposure technique parameters for acquiring mammographic images of a patient based on a set of patient data (e.g., non-training data). The set of patient data may comprise data that is similar in type and/or value to the training data. For example, the set of patient data may comprise at least patient data and imaging data for a patient. At least a portion of the set of patient data may be collected from a patient during a current patient visit.

Using the set of patient data, the ML model may output optimal exposure technique parameters for the patient in real-time. The optimal exposure technique parameters output by the AI model may then be used to generate one or more mammographic images of the patient in real-time. For example, during a current patient visit, an ML model may receive a set of patient data and output exposure technique parameters. The exposure technique parameters may be used to generate mammographic images during the patient visit. In some aspects, the output of the ML model may be compared to the parameters of the traditional method for performing AEC. A decision regarding the output exposure technique parameters to be implemented during the mammographic imaging may then be made based on the output of the ML model, the parameters for performing the traditional AEC method, or a combination thereof.

Accordingly, the present disclosure provides a plurality of technical benefits including but not limited to: minimizing radiation overexposure to patient during medical imaging, reducing occurrences of underexposure during medical imaging, optimizing the image quality of medical images, providing patient-specific exposure technique prediction, eliminating the need for a scout image during medical imaging, and evaluating ML-based exposure technique parameters using parameters for traditional AEC methods, among others.

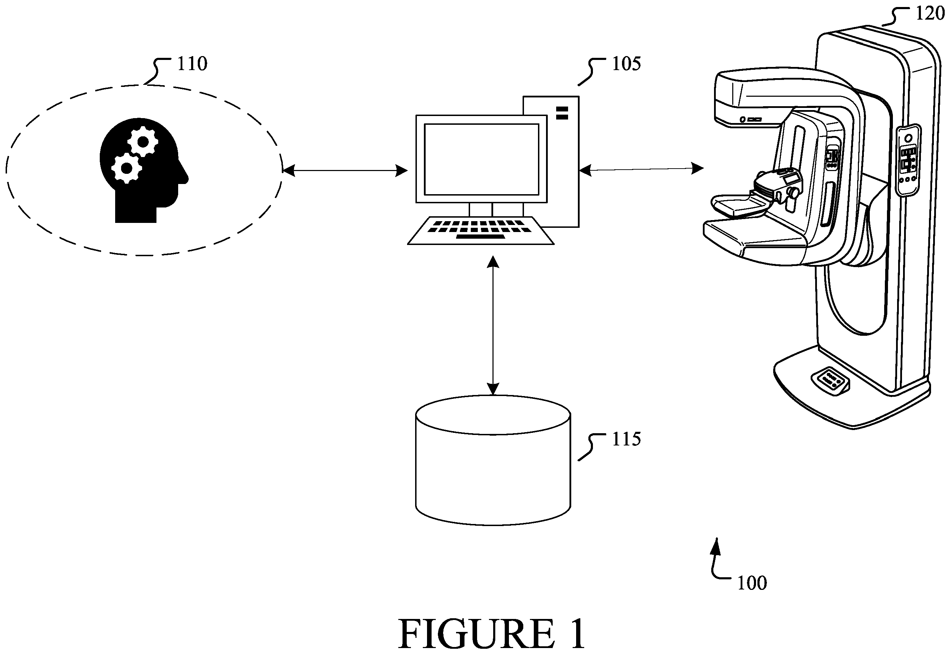

Figure 1 illustrates an overview of an example system for using ML to predict optimal exposure technique for acquiring mammographic images. Example system 100 as presented is a combination of interdependent components that interact to form an integrated system. System 100 may comprise hardware components and/or software components implemented on and/or executed by hardware components. System 100 may provide one or more operating environments for software components to execute according to operating constraints, resources, and facilities of system 100. In some examples, the operating environment(s) and/or software components may be provided by a single processing device, as depicted in Figure 6. In other examples, the operating environment(s) and software components may be distributed across multiple devices. For instance, input may be entered on a user device and information may be processed or accessed using other devices in a network, such as one or more network devices and/or server devices.

In aspects, system 100 may represent a computing environment comprising sensitive or private information associated with, for example, a healthcare facility, healthcare patients, and/or healthcare personnel. Although specific reference to a healthcare environment is described herein, it is contemplated that the techniques of the present disclosure may be practiced in other environments. For example, system 100 may represent a software development environment or an alternative environment that does not comprise sensitive or private medical information.

In Figure 1, system comprises computing device 105, ML component 110, datastore(s) 115, and medical device 120. One of skill in the art will appreciate that the scale of systems such as system 100 may vary and may include more or fewer components than those described in Figure 1. As one example, the functionality and/or one or more components of computing device 105 may be integrated into medical device 115. As another example, the functionality and/or one or more components of intelligence component 110 may be integrated into computing device 105.

Computing device 105 may be configured to receive or collect input from one or more users or devices. Computing device 105 may include sensors, applications, and/or services for receiving or collecting input. Example sensor include microphones, touch-based sensors, keyboards, pointing/selection tools, optical/magnetic scanners, accelerometers, magnetometers, gyroscopes, etc. The collected input may include, for example, voice input, touch input, text-based input, gesture input, video input, and/or image input. For example, computing device 105 may enable a user, such as healthcare professional, to input and/or retrieve medical data (e.g., patient records, medical and treatment history information, patient health data, healthcare professional notes and documents, medical images, medical procedure parameters, medical equipment information). The medical data retrieved by computing device 105 may be retrieved from and/or stored in one or more data stores, such as data store(s) 115. Computing device 105 may also be configured to store, execute, transmit and/or provide access to one or more predictive or statistical models or algorithms. For example, computing device 105 may provide access to an ML model for determining optimal exposure technique parameters for acquiring mammographic images of a patient. Examples of computing device 105 include, but are not limited to, personal computers (PCs), server devices, mobile devices (e.g., smartphones, tablets, laptops, personal digital assistants (PDAs)), and wearable devices (e.g., smart watches, smart eyewear, fitness trackers, smart clothing, body-mounted devices).

ML component 110 may be configured to train one or more predictive or statistical models or algorithms. In aspects, ML component 110 may receive a request to train a model or to provide a trained model. The request may comprise training data, such as patient attribute data, image metadata, image pixel data, exposure technique parameters, etc. Alternatively, the request may comprise model selection information, such as an identifier, a description, a keyword, a requested task, etc. Upon receiving the request, ML component 110 may select a model or a model template that matches the request. The model or model template may be selected from a model repository (not pictured) that stores one or more model templates and/or trained models. Alternatively, ML component 110 may generate a model or a model template that matches the request. ML component 110 may then train the model or a model template as defined in the request. As a specific example, ML component 110 may train a model template to determine the optimal exposure technique parameters for acquiring mammographic images of a patient.

In examples, ML component 110 may represent a computing device, a software component of a computing device, or multiple software components that are distributed across multiple computing devices. In some examples, ML component 110 and computing device 105 may be implemented in the same computing environment. For instance, ML component 110 and computing device 105 may be located in a healthcare computing environment comprising sensitive medical data. In other examples, ML component 110 and computing device 105 may be implemented in separate computing environments. For instance, computing device 105 may be located in a healthcare computing environment and ML component 110 may be located in a software development environment that is geographically and/or logically separated from the healthcare computing environment.

Data store(s) 115 may be configured to store medical data and/or other types of data and information. For example, data store(s) 115 may comprise patient data (e.g., breast thickness, breast density, breast size, implant/foreign object identifiers, patient vital statistics, patient treatment history, and other patient-related information), imaging data (e.g., patient images, reference images, pixel data, DICOM information, imaging mode options, paddle type) and/or exposure technique parameters for one or more images or imaging modalities (e.g., kilovoltage peak (kVp), milliamp-seconds (mAs), X-ray filter combination, focal spot information, source-to-image receptor distance, object-to-image receptor distance, scatter grid information, beam restriction information). Data store(s) 115 may also comprise one or more models, algorithms, or templates thereof. For instance, a model or algorithm provided by ML component 110 may be stored in data store(s) 115. Examples of data store(s) 115 include, but are not limited to, databases, file systems, directories, flat files, and email storage systems. In aspects, data store(s) 115 may be accessible to one or more components of system 100. For example, one or more of data store(s) 115 may be located in, or accessible from, the computing environments of computing device 105 and/or ML component 110.

Medical device 120 may be configured to generate or collect images and/or physical attribute data of a patient. In aspects, medical device 120 may comprise one or more sensor components, such as image sensors, depth sensors, tracking sensors, proximity sensors, stereo/HD cameras, and infrared cameras. The sensor components may be used to collect data and/or images relating to physical attributes of the patient, such as breast size, breast thickness, breast volume, and/or breast shape. Although specific references to mammography is described herein, it is contemplated that the techniques of the present disclosure may be practiced with alternative types of imaging. Examples of medical device 120 include, but are not limited to, ultrasound devices, CT devices, X-ray device, magnetic resonance imaging (MRI) devices, and positron-emission tomography (PET) devices.

Medical device 120 may be further configured to store and/or execute one or more models. For example, medical device 120 may receive a trained model from ML component 110 or computing device 105. Medical device 120 may store the trained model locally and/or transmit the trained model to one or more other devices. Medical device 120 may execute the trained model (or cause the trained model to be executed by another device) in response to one or more events. As one example, during a patient visit, a healthcare professional may position a patient on/in medical device 120. Medical device 120 may receive/collect patient data (e.g., breast thickness, breast density, breast size, implant/foreign object identifiers) associated with the patient and/or imaging data (e.g., pixel data, imaging mode, paddle type) associated with an imaging procedure to be performed on the patient. Based on the received/collected patient data and/or imaging data, medical device 120 may execute the trained model. For instance, medical device 120 may provide the patient data and/or imaging data as input parameters to the trained model. The trained model may output a prediction of the optimal exposure techniques for acquiring mammographic images of the patient. Medical device 120 may then execute an imaging procedure on the patient based on the optimal exposure techniques output by the trained model.

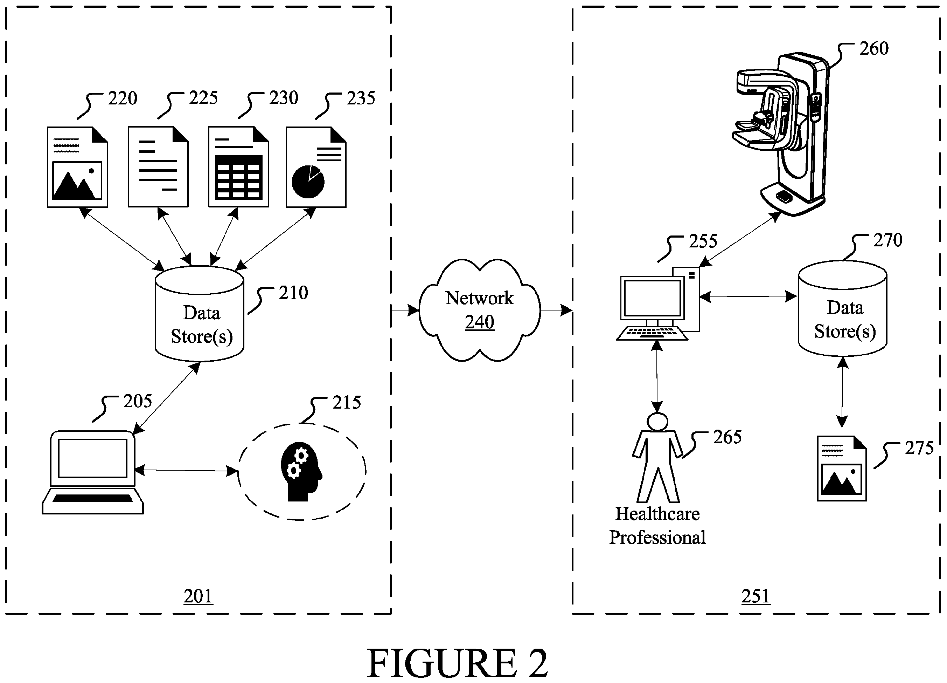

Figure 2 is a diagram of an example process flow for using ML to predict optimal exposure technique for acquiring mammographic images. Process flow 200, as presented, comprises environments 201 and 251. In examples, environment 201 may represent a software development environment or a third-party environment. Environment 201 may be web-based, cloud-based, or otherwise implemented remotely from environment 251. Environment 201 may be publicly or selectively accessible and may implement security procedures to enable the secure access of environment 251. Generally, environment 201 may not store or have access to sensitive or private information comprised by environment 251. Environment 251 may represent a healthcare facility, such as a hospital, an imaging and radiology center, an urgent care facility, a medical clinic or medical offices, an outpatient surgical facility, a physical rehabilitation center, etc. Environment 251 may comprise sensitive or private information associated with a healthcare facility, healthcare patients, and/or healthcare personnel. Environments 201 and 251 may be physically and/or logically separated. In addition, environments 201 and 251 may be separated by firewalls and authentication protocols ensuring safe handling of the sensitive medical information comprised in environment 251. One of skill in the art will appreciate that the number and type of environments and/or components associated with environments 201 and 251 may vary from those described in Figure 2.

In examples, environment 201 may comprise training device 205, data store(s) 210, and ML component 215. Training device 205 may be configured to receive data and/or instructions from one or more users or devices. For example, a user, such as a software developer or a healthcare professional, may directly access training device 205 from environment 201. Alternatively, a user may remotely access training device 205 from a computing environment other than environment 201. The data/instructions received by training device 205 may correspond to a request to train a model or to provide a trained model. In one example, the request may comprise training data, such as patient attribute data, image metadata, image pixel data, exposure technique parameters, etc. In another example, the request may comprise information for identifying the location of training data. In yet another example, the request may comprise information for selecting a model or a model type, such as an identifier, a description, a keyword, a requested task, etc. Training device 205 may use the received data/instructions to search one or more data stores, such as data store(s) 210, for relevant training data.

Data store(s) 210 may comprise at least mammogram images 220, equipment data 225, patient data 230, and exposure technique parameters 235. Mammogram images 220 may represent images determined to be of optimal image quality (e.g., optimal optical density, signal-to-noise ratio, contrast-to-noise ratio, mean square error, contrast improvement ratio). The classification of image quality (e.g., high quality, moderate quality, low quality) may be a manual determination, an automated determination, or some combination thereof. For example, a user may manually review a set of images, classify the image qualities of the respective images, and/or provide annotations indicating the image qualities. Alternatively, one or more supervised and/or unsupervised ML techniques may be used to review, classify, and/or annotate the set of images.

Equipment data 225 may represent data and parameter settings associated with equipment used during medical imaging. For example, equipment data 225 may include information relating to imaging device type (e.g., X-ray device, CT device, MRI device, ultrasound device), compression paddle type (e.g., paddle size, paddle shape, paddle flexibility, paddle padding, paddle type), imaging modes (e.g., 2D, 3D, tomography), anode material (e.g., tungsten, rhodium, molybdenum), and imaging filter type (e.g., rhodium, silver, aluminum), among others. Patient data 230 may represent patient attribute information. For example, patient data 230 may include patient characteristics (e.g., breast size, breast thickness, breast density), foreign object identification (e.g., breast implants, biopsy markers, pacemakers), and general medical information (e.g., patient identification, vitals, condition(s), treatment(s)), among others. Exposure technique parameters 235 may represent the various exposure technique parameters used during imaging. For example, exposure technique parameters 235 may include kVp, mAs, X-ray filter combination, focal spot information, source-to-image receptor distance, object-to-image receptor distance, scatter grid information, beam restriction information.

In examples, the data in data store(s) 210 may be stored such that equipment data 225, patient data 230, and exposure technique parameters 235 are correlated to respective mammogram images 220. As one example, a data structure (e.g., a database, a table, a spreadsheet) may be used to correlate the equipment data, patient data, and/or exposure technique associated with a mammographic image. Alternatively, each mammogram image may comprise or be annotated with the corresponding equipment data, patient data, and/or exposure technique parameters associated with the creation of that mammographic image. For instance, the equipment data, patient data, and/or exposure technique parameters may be stored in the DICOM header (or other areas) of the mammographic image.

In aspects, training device 205 may provide the training data received from data store(s) 210 to ML component 215. ML component 215 may use the training data to train one or more predictive or statistical models or algorithms. For example, one or more portions of mammogram images 220, equipment data 225, patient data 230, and exposure technique parameters 235 may be provided as input to ML component 215. Based on the input, ML component 215 may identify the exposure technique parameters, patient attributes, and equipment parameters that are associated with optimal image quality. ML component 215 may use the input as target values to train a model for determining the optimal exposure technique parameters for acquiring mammographic images of various patients. ML component 215 may provide the trained model to training device 205, data store(s), and/or a model repository (not pictured) that stores one or more model templates and/or trained models. Training device 205 or ML component 215 may then transmit the trained model to environment 251 via network 240. Examples of network 204 include, but are not limited to, personal area networks (PANs), local area networks (LANs), metropolitan area networks (MANs), and wide area networks (WANs).

In examples, environment 251 may comprise computing device 255, medical device 260, healthcare professional 265, and data store(s) 270. Computing device 255 may receive a trained model from environment 201. Computing device 255 may be configured as discussed with respect to computing deice 105 of Figure 1. Computing device 255 may store the trained model locally and/or transmit the trained model to one or more medical devices, such as medical device 260. Medical device 260 may store the trained model locally. Medical device 260 may be configured as discussed with respect to medical device 120 of Figure 1.

In aspects, the trained model may be invoked during an interaction associated with a patient or patient data. As one specific example, during a patient visit to environment 251, a healthcare professional, such as healthcare professional 265 may use computing device 255 to initiate the setup process for performing an exposure. Healthcare professional 265 may represent, for example, a radiologist, a surgeon or other physician, a technician, a practitioner, or someone acting at the behest thereof. During the setup process, healthcare professional 265 may select an exposure method to use. For instance, healthcare professional 265 may choose between using the traditional AEC method and the trained model. Alternatively, the determination may be made automatically based on one or more factors, such as available medical devices, the presence of a patient in/on a medical device, patient data, etc.

After an exposure method has been selected, patient data may be collected from the patient by healthcare professional 265. For instance, healthcare professional 265 may solicit general medical information and information regarding implanted foreign objects from the patient. Patient data may also be collected from the patient by medical device 260. For instance, during (or prior to) the setup process, healthcare professional 265 may position a patient on/in medical device 260. While the patient is positioned on/in medical device 260, medical device 260 may be used to perform a procedure to collect patient characteristics, such as breast thickness or breast density, from the patient. Healthcare professional 265 and/or medical device 260 may provide the patient data to computing device 255. Medical device 260 may also provide equipment data of medical device 260 to computing device 255.

Upon receiving the patient data and/or equipment data, computing device 255 may use one or more parsing and/or pattern matching techniques to identify search information (e.g., keywords, timestamps, or other information relating to the patient) in the patient data and/or equipment data. The search information may be used to search one or more data stores, such as data store(s) 270. Data store(s) 270 may comprise at least image data 275. In examples, data store(s) 270 may represent a medical imaging technology, such as picture archiving and communication system (PACS) or radiology information system (RIS). Mammogram images 275 may represent images and image data for one or more patients. In aspects, image data associated with the patient may be collected from data store(s) 270. For example, computing device 255 may collect previously collected mammographic images for the patient from data store(s) 270.

Computing device 255 may provide the patient data, the equipment data, and/or the image data associated with the patient as input to the trained model. Based on the input, the trained model may output predicted optimal exposure technique parameters specific to the patient. For example, the trained model may provide the kVp, mAs, and X-ray filter combination parameters for acquiring mammographic images of optimal image quality. The predicted optimal exposure technique parameters may then be used to perform the exposure and acquire the mammographic images of the patient.

In some aspects, prior to performing the exposure, the predicted optimal exposure technique parameters may be compared to the parameters of the traditional method for performing AEC (e.g., sampling a patient's breast and searching for the densest regions of the breast). The comparison may made using a ML component (not pictured) of or accessible to computing device 255. The ML component may implement, for example, decision trees, logistic regression, support vector machines (SVM), k-nearest-neighbor (KNN) algorithms, neural networks, Naive Bayes classifiers, linear regression, k-means clustering, or the like. In one example, the ML component may combine the parameters predicted using the trained model and the parameters for the traditional AEC method into a single set of parameters. For instance, the two sets of parameters may be averaged. Alternatively, weights may be applied to one or more values in each set of parameters and the values may be combined (e.g., via addition, multiplication, dot product).

In another example, the ML component may use one of the sets of parameters to identify and remove statistical outlying values from the other set of parameters. For instance, the parameters predicted using the trained model may be compared to the parameters for the traditional AEC method. Any value in the parameters predicted using the trained model that is more than a predefined percentage (e.g., ±20%) away from a corresponding value in the parameters for the traditional AEC method may be removed or substituted for the value in the parameters for the traditional AEC method. In aspects, the set of parameters resulting from the comparison of the parameters predicted using the trained model and the parameters for the traditional AEC method may be used to perform the exposure and acquire the mammographic images of the patient. The set of parameters resulting from the comparison may enable the acquisition of the mammographic images having an optimal image quality and/or enable an optimal radiation dose to be delivered to the patient.

Having described a system and process flow that may employ the techniques disclosed herein, the present disclosure will now describe one or more methods that may be performed by various aspects of the present disclosure. In aspects, methods 300 and 400 may be executed by a system, such as system 100 of Figure 1. However, methods 300 and 400 are not limited to such examples. In other aspects, methods 300 and 400 may be performed by a single device comprising multiple computing environments. In at least one aspect, methods 300 and 400 may be executed by one or more components of a distributed network, such as a web service/distributed network service (e.g. cloud service).

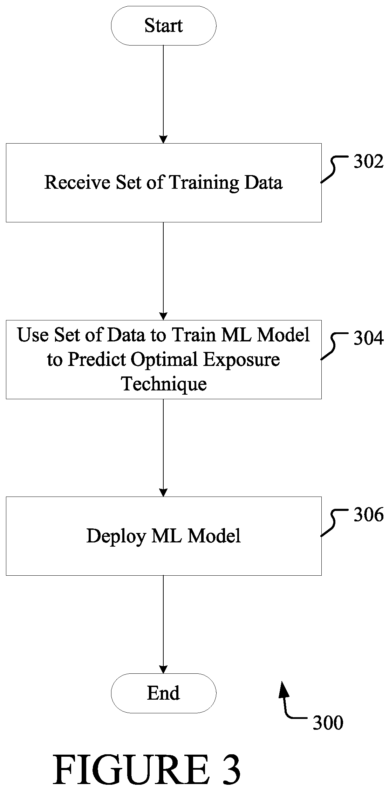

Figure 3 illustrates an example method for training an ML model for predicting optimal exposure technique for acquiring mammographic images. Example method 300 may be executed by a user, such as a software developer, in a computing environment, such as environment 201. Example method 300 begins at operation 302, where a dataset is received from one or more data stores. In aspects, a computing device, such as training device 205, may be used to access a data store, such as data store(s) 210. The computing device may a dataset to be received or collected from the data store. As a specific example, the computing device may comprise a user interface that is accessible by a user. The user may enter a search query, such as "exposure technique parameter model," into the user interface. The user may also select one or more menu options provided by the user interface to refine the selected dataset and achieve a desired dataset scope. For instance, the menu options may enable the user to select specific parameters, such as breast type, compression paddle type, presence of foreign objects, foreign object type, imaging mode, X-ray filter combination, etc. The search query and search query parameters may be used to collect a corresponding dataset from the data store.

The dataset received from the data store may comprise mammographic images, equipment data, patient data, and/or exposure technique parameters for a set of patients. The mammographic images may represent images that are of optimal or high image quality. Each mammographic image may be correlated to a corresponding set of equipment data, patient data, and/or exposure technique parameters. For instance, a DICOM header of the mammographic image may comprise at least a portion of the equipment data, patient data, and/or exposure technique parameters associated with that mammographic image. In examples, the dataset may comprise training data that is unlabeled and/or labeled using, for example, text indicators, numerical values, highlighting, encircling (and/or other types of content enclosing), arrows or pointers, font or style modifications, or other forms of annotation.

At operation 304, the dataset is used to train an ML model. In aspects, the dataset may be provided as input to a ML mechanism, such as ML component 215. The ML mechanism may use the dataset to train one or more predictive or statistical models or algorithms. For example, based on the dataset, ML mechanism may train a predictive model for determining the optimal exposure technique parameters (e.g., mAs, kVp, X-ray filter combination) for acquiring mammographic images of one or more patients. The training may comprise assigning scores and/or weights to one or more parameters in the dataset. In examples, a higher score/weight may be assigned to a parameter or a type of parameter in the dataset to indicate that the parameter/type of parameter has a larger effect on the exposure technique parameters output by the model. During training of the predictive model, the scores/weights may be adjusted until the predictive model outputs predicted exposure technique parameters that are within a predefined range or relate to one or more pre-determined target values. The predefined range or target values may correspond to a radiation exposure range in which sufficient image quality is obtainable and excessive radiation exposure does not occur.

At operation 306, the ML model is deployed to one or more devices. In aspects, the trained ML model may be stored in one or more storage locations. For example, the trained ML model may be stored on the computing device, in the data store, or in a model repository that stores one or more model templates and/or trained models. One or more of storage locations may be located in a public and/or development environment, such as environment 251. In some aspects, the trained ML model may also (or alternatively) be deployed to one or more devices in an environment comprising sensitive or private information, such as environment 201. For example, the trained ML model may be deployed to a medical workstation, such as computing device 255, or medical equipment, such as medical device 260.

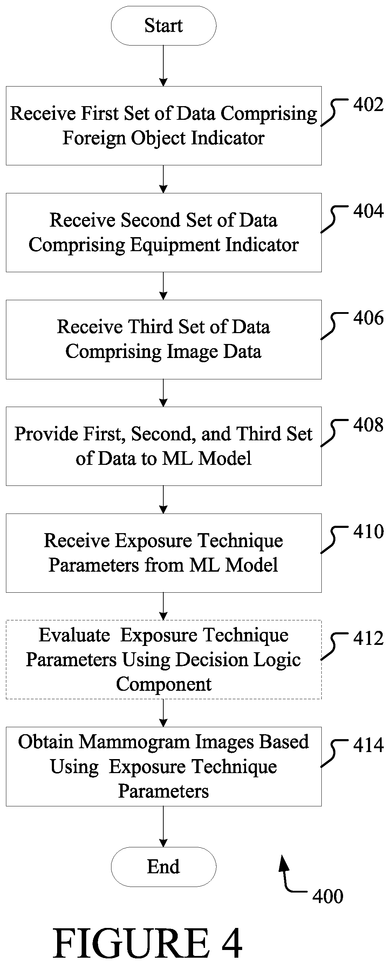

Figure 4 illustrates an example method for using ML to predict optimal exposure technique for acquiring mammographic images. Example method 400 may be executed by a user, such as a healthcare provider, in a computing environment, such as environment 251. In examples, example method 400 may be initiated by a healthcare professional as part of an imaging procedure during a patient visit to a healthcare facility. However, example method 400 is not limited to such an example.

Example method 400 begins at operation 402, where a first set of data is received. In aspects, a computing device, such as computing device 255, may receive a first set of data from one or more sources. The first set of data may comprise patient data of the patient, such as patient breast attributes (e.g., such as thickness, density, size), patient positioning information (e.g., bilateral craniocaudal (CC) view, mediolateral oblique (MLO) view, true lateral view), general medical information, information regarding foreign objects within the patient (e.g., breast implants, biopsy markers, pacemakers), and other types of patient-related information. Collecting this patient specific data for the particular patient being imaged may be relevant for determining the exposure technique parameters, as the presence of breast implants, biopsy markers, and pacemakers, etc. may impact the optimal amount of exposure necessary to obtain high quality images without overexposing the patient. As could be appreciated, the presence of implant and different types of implants, made of different materials may require different amount of exposure. For example, a healthcare professional, such as healthcare professional 265, may input patient data solicited from a patient into the computing device. Alternatively, the patient or a user device of the patient may input or otherwise provide the patient data to the computing device. For instance, a wearable device of the patient may automatically upload the patient data to the computing device when the patient comes within a certain range of the computing device or the healthcare facility.

At operation 404, a second set of data is received. In aspects, medical equipment, such as medical device 260, may provide a second set of data to the computing device. The second set of data may also comprise patient data of the patient, such as breast thickness and/or breast density. For example, a patient may be positioned on/in medical equipment, such as a mammography unit. The mammography unit may be communicatively coupled (or couplable) to the computing device. The mammography unit may comprise a compression component, such as a compression arm and/or a compression paddle, for compression of a patient breast during and/or prior to imaging procedures. The compression component may apply a compression to the patient's breast to determine a thickness value for the breast. The mammography unit may provide the thickness value to the computing device. Collecting the breast thickness, density data, and/or patient positioning information for the particular patient being imaged may be relevant for determining the optimal amount of exposure necessary to obtain high quality images without overexposing the patient. For example, a higher amount of exposure may be required for breasts having a high thickness or density and a lower amount of exposure may be required for breasts having a lower thickness or density.

The second set of data may also comprise equipment data of the medical equipment, such as imaging device attributes (e.g., imaging device type, model, capabilities), compression component attributes (e.g., compression paddle size, shape, flexibility, padding, type), imaging modes (e.g., 2D, 3D, tomography), anode material (e.g., tungsten, rhodium, molybdenum), and imaging filter type (e.g., rhodium, silver, aluminum). Collecting medical equipment data used for the specific exam may be relevant for determining the exposure technique parameters, as the use of different paddles having different thicknesses, sizes and shapes, as well as the imaging modes, and filter types, etc. may impact the optimal amount of exposure necessary to obtain high quality images without overexposing the patient. For example, some paddles may be made of thicker material and would potentially require additional exposure than other paddles made of thinner material. Similarly, different imaging modes require different amount of exposure. In addition, different mammography devices or models may require different amount of exposure. The amount of exposure optimally needed for the type of equipment may be stored in a look up table associated with the particular equipment types.

At operation 406, a third set of data is received. In aspects, the computing device may receive a third set of data from one or more data stores, such as data store(s) 270. The third set of data may comprise prior image data of the patient, such as previously collected medical images (e.g., mammogram images), pixel data, exposure technique parameters, DICOM header information, and data associated with the medical images (e.g., breast density values, breast thickness values, biopsy implant flag). For example, in response to receiving the first set of data and/or the second set of data, the computing device may collect one or more mammogram images collected from the patient during previous patient visits. The previously collected mammogram images may be identified by matching patient identifying information in the first set of data and/or the second set of data, such as patient name or identifier, with patient information in the DICOM header of the mammogram images. Collecting this prior imaging data for the particular patient may be relevant for determining the exposure technique parameters. For example, data on breast density values or breast thickness values may not be in the first set of data received but may impact the determination of the exposure technique parameters. By taking into account prior imaging data for the patient, an optimal amount of exposure necessary to obtain high quality images without overexposing the patient may be determined. As described above and could be appreciated, there may be a mathematical relationship between breast thickness values and/or breast density values and the amount of exposure necessary.

At operation 408, the first set of data, the second set of data, and the third set of data are provided to an ML model, such as ML component 215. In aspects, the computing device and/or the medical equipment may have access to an ML model that is trained to predict optimal exposure technique for acquiring mammographic images. The ML model may be stored on the computing device, on the medical equipment, in the data store, or in a model repository that stores one or more model templates and/or trained models. The storage locations of the ML model may be in a public and/or development environment (e.g., environment 251), in an environment comprising sensitive or private information (e.g., environment 201), or in a combination thereof. The healthcare professional may invoke the ML model using the computing device, for example, by selecting a setting or a button on a user interface associated with the mammography system. In one such example, the healthcare processional may select the ML model feature after positioning the patient but prior to imaging the patient. As the input is received in the system as described above. Alternatively, the ML model may be invoked automatically upon the occurrence of one or more events. As a specific example, the ML may automatically be invoked when the first set of data is received by the computing device, the patient is positioned in the medical equipment, the second set of data is received by the computing device, or the third set of data is received by the computing device. Once invoked, one or more portions of the first set of data, the second set of data, and/or third set of data may be provided as input to the ML model.

At operation 410, a set of exposure technique parameters may be received from the ML model. In aspects, the ML model may output a set of exposure technique parameters that are specific to the patient, such as kVp values, mAs values, X-ray filter combination information, focal spot information, source-to-image receptor distance values, object-to-image receptor distance values, scatter grid information, and beam restriction information. The set of exposure technique parameters may represent the optimal exposure technique parameters for acquiring mammographic images of the patient based on patient-specific factors that are known to affect the exposure technique parameters. The set of exposure technique parameters may be provided to the computing device and/or the medical equipment. The set of exposure technique parameters may also be stored in one or more data stores, such as data store(s) 270.

At optional operation 412, the set of exposure technique parameters may be evaluated by a decision logic component. In aspects, the set of exposure technique parameters may be provided to (or otherwise made accessible to) a decision logic component, such as the ML component discussed with respect to Figure 2. The decision logic component may compare the set of exposure technique parameters to a separate set of exposure technique parameters. For example, the set of exposure technique parameters may be compared to a set of parameters for the traditional method for performing AEC ("traditional AEC parameters"). The traditional AEC parameters may be provided by the healthcare professional or received/collected from a different source. The decision logic component may apply one or more scores/weights to the parameters in each set of parameters and/or combine the scored/weighted set of parameters into a single, combined set of exposure technique parameters. Alternatively, the decision logic component may use one of the sets of parameters to identify and remove statistical outlying values from the other set of parameters. For example, decision logic component may use the traditional AEC parameters to define an expected/acceptable range of values for the set of exposure technique parameters. Removing the statistical outlying values from the set of exposure technique parameters may result in a modified set of exposure technique parameters.

At operation 414, one or more mammogram images of the patient are obtained. In aspects, a set of exposure technique parameters may be used as input parameters for an imaging procedure performed on the patient in real-time (e.g., during the patient visit). The imaging procedure may be performed by the medical equipment and/or other medical devices. The imaging procedure may generate one or more mammogram images and/or other types of medical images of the patient. The radiation exposure of the patient during the imaging procedure may be defined by the set of exposure technique parameters. The set of exposure technique parameters may represent the minimal exposure to the patient that is necessary to acquire optimal mammographic images of the patient. By using the exposure technique parameters determined herein, the need for a scout image and the associated exposure to the patient may be eliminated.

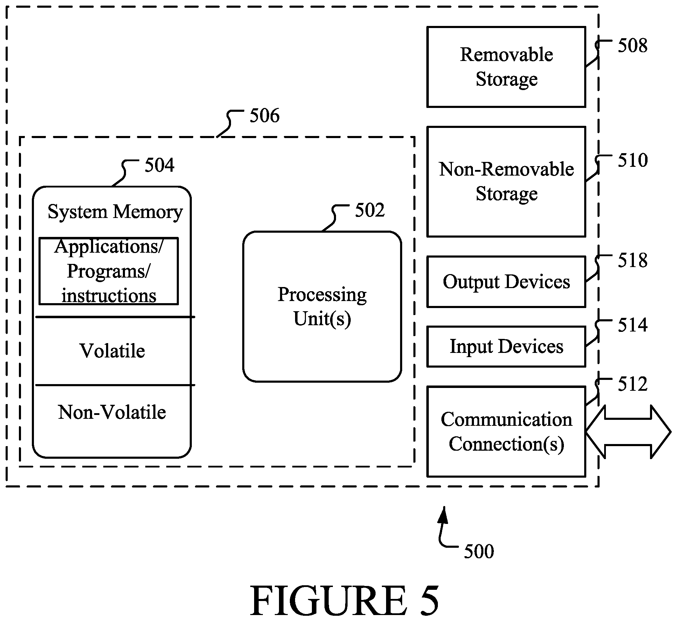

Figure 5 illustrates an exemplary suitable operating environment for the automating clinical workflow decision techniques described in Figure 1. In its most basic configuration, operating environment 500 typically includes at least one processing unit 502 and memory 504. Depending on the exact configuration and type of computing device, memory 504 (storing, instructions to perform the techniques disclosed herein) may be volatile (such as RAM), non-volatile (such as ROM, flash memory, etc.), or some combination of the two. This most basic configuration is illustrated in Figure 5 by dashed line 506. Further, environment 500 may also include storage devices (removable, 508, and/or non-removable, 510) including, but not limited to, magnetic or optical disks or tape. Similarly, environment 500 may also have input device(s) 514 such as keyboard, mouse, pen, voice input, etc. and/or output device(s) 516 such as a display, speakers, printer, etc. Also included in the environment may be one or more communication connections 512, such as LAN, WAN, point to point, etc. In embodiments, the connections may be operable to facility point-to-point communications, connection-oriented communications, connectionless communications, etc.

Operating environment 500 typically includes at least some form of computer readable media. Computer readable media can be any available media that can be accessed by processing unit 502 or other devices comprising the operating environment. By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media includes, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other non-transitory medium which can be used to store the desired information. Computer storage media does not include communication media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, microwave, and other wireless media. Combinations of the any of the above should also be included within the scope of computer readable media.

The operating environment 500 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media. Such networking environments are commonplace in offices, enterprise-wide computer networks, intranets and the Internet.

The embodiments described herein may be employed using software, hardware, or a combination of software and hardware to implement and perform the systems and methods disclosed herein. Although specific devices have been recited throughout the disclosure as performing specific functions, one of skill in the art will appreciate that these devices are provided for illustrative purposes, and other devices may be employed to perform the functionality disclosed herein without departing from the scope of the disclosure.

This disclosure describes some embodiments of the present technology with reference to the accompanying drawings, in which only some of the possible embodiments were shown. Other aspects may, however, be embodied in many different forms and should not be construed as limited to the embodiments set forth herein. Rather, these embodiments were provided so that this disclosure was thorough and complete and fully conveyed the scope of the possible embodiments to those skilled in the art.

Although specific embodiments are described herein, the scope of the technology is not limited to those specific embodiments. One skilled in the art will recognize other embodiments or improvements that are within the scope of the present technology. Therefore, the specific structure, acts, or media are disclosed only as illustrative embodiments. The scope of the technology is defined by the following claims.

## Claims

1. A system (100) comprising:
a processor; and
memory coupled to the processor, the memory comprising computer executable instructions that, when executed, perform a method comprising:
receiving (402) a first data set comprising patient-specific data of a patient;
receiving (404) a second data set comprising an equipment-specific information, the equipment-specific information indicating attributes or accessories of an imaging device;
receiving (406) a third data set comprising prior image data of the patient wherein the prior image data comprises one or more mammogram images of the patient, the one or more mammogram images collected during a previous patient visit;
providing (408) the first data set, the second data set, and the third data set as input to a machine learning model;
receiving (410) a set of exposure technique parameters as output from the machine learning model, the set of exposure technique parameters comprising at least one of: a kilovoltage peak (kVp) value, a milliamp-seconds (mAs) value, or an X-ray filter combination; and
acquiring (414) an image of the patient based on the set of exposure technique parameters.

2. The system of claim 1, wherein, prior to acquisition of the image, a user invokes the machine learning model.

3. The system of any of claims 1-2, wherein the patient-specific data includes a foreign object indicator, the foreign object indicator indicating whether a foreign object is implanted in a breast of a patient.

4. The system of any of claims 1-3, wherein:
a healthcare professional provides the first data set to a computing device used to train the machine learning model;
a medical device provides the second data set to the computing device; and
a data store provides the third data set to the computing device.

5. The system of any of claims 1-4, wherein the first data set further comprises at least one of: thickness of the breast, density of the breast, or patient positioning information.

6. The system of any of claims 1-5, wherein attributes of an imaging device comprise at least one of: compression paddle size, compression paddle shape, compression paddle type, imaging mode, anode material, or imaging filter type.

7. The system of any of claims 1-6, wherein the first data set and the second data set are collected during a current patient visit to a healthcare facility,
and/or
wherein the third data set further comprises at least one of thickness of the breast or density of the breast.

8. The system of any of claims 1-7, wherein the machine learning model is trained to predict optimal exposure technique for acquiring mammographic images.

9. The system of any of claims 1-8, wherein set of exposure technique parameters further comprises at least one of: focal spot information, source-to-image receptor distance values, object-to-image receptor distance values, scatter grid information, or beam restriction information.

10. A method (400) comprising:
receiving (402), at a computing device (105), a first data set comprising patient-specific data of a patient;
receiving (404), at the computing device (105), a second data set comprising an equipment-specific information, the equipment-specific information indicating attributes or accessories of an imaging device;
receiving (406), at the computing device (105), a third data set comprising prior image data of the patient wherein the prior image data comprises one or more mammogram images of the patient, the one or more mammogram images collected during a previous patient visit;
providing (408), by the computing device, the first data set, the second data set, and the third data set as input to a machine learning model;
receiving (410) a set of exposure technique parameters as output from the machine learning model, the set of exposure technique parameters comprising at least one of: a kilovoltage peak (kVp) value, a milliamp-seconds (mAs) value, or an X-ray filter combination; and
generating (414) one or more mammogram images of the patient based on the set of exposure technique parameters, the one or more mammogram images being generated by the imaging device.

11. The method of claim 10, wherein:
the imaging device is communicatively couplable to the computing device; and
the imaging device provides the second data set to the computing device when the imaging device and the computing device are communicatively coupled.

12. The method of any of claims 10-11, wherein the one or more mammogram images are of an optimal image quality based on at least one of: optical density, signal-to-noise ratio, contrast-to-noise ratio, mean square error, contrast improvement ratio.

13. The method of any of claims 10-12, wherein the method further comprises:
evaluating the set of exposure technique parameters using a machine learning component of the computing device, the evaluation comprising comparing the set of exposure technique parameters to a set of parameters for a traditional automatic exposure control (AEC) method.

14. The method of claim 13, wherein:
the comparing comprises combining the set of exposure technique parameters with the set of parameters for the traditional AEC method to generate a combined set of exposure technique parameters; and
the method further comprises:
using the combined set of exposure technique parameters to generate a set of mammogram images of the patient.

15. The method of claim 13, wherein:
the comparing comprises using the set of parameters for the traditional AEC method to identify statistical outlying values in the set of exposure technique parameters.

## Patentansprüche

1. System (100), umfassend:
einen Prozessor; und
Speicher, der an den Prozessor gekoppelt ist, wobei der Speicher computerausführbare Anweisungen umfasst, die bei Ausführung ein Verfahren durchführen, das Folgendes umfasst:
Empfangen (402) eines ersten Datensatzes, der patientenspezifische Daten eines Patienten umfasst;
Empfangen (404) eines zweiten Datensatzes, der gerätespezifische Informationen umfasst, wobei die gerätespezifischen Informationen Attribute oder Zubehör einer Bildgebungsvorrichtung angeben;
Empfangen (406) eines dritten Datensatzes, der frühere Bilddaten des Patienten umfasst, wobei die früheren Bilddaten ein oder mehrere Mammographiebilder des Patienten umfassen, wobei die ein oder mehreren Mammographiebilder während eines vorherigen Patientenbesuchs gesammelt wurden;
Bereitstellen (408) des ersten Datensatzes, des zweiten Datensatzes und des dritten Datensatzes als Eingabe für ein Maschinenlernmodell;
Empfangen (410) eines Satzes von Belichtungstechnikparametern als Ausgabe von dem Maschinenlernmodell, wobei der Satz von Belichtungstechnikparametern wenigstens eines hiervon umfasst: einen Kilovolt-Spitzenwert (kVp), einen Milliamperesekundenwert (mAs) oder eine Röntgenfilterkombination; und
Erfassen (414) eines Bildes des Patienten basierend auf dem Satz von Belichtungstechnikparametern.

2. System nach Anspruch 1, wobei, vor Erfassung des Bildes, ein Benutzer das Maschinenlernmodell aufruft.

3. System nach einem der Ansprüche 1-2, wobei die patientenspezifischen Daten einen Fremdkörperindikator beinhalten, wobei der Fremdkörperindikator angibt, ob ein Fremdkörper in einer Brust eines Patienten implantiert ist.

4. System nach einem der Ansprüche 1-3, wobei:
eine Gesundheitsfachkraft den ersten Datensatz für eine Rechenvorrichtung bereitstellt, die verwendet wird, um das Maschinenlernmodell zu trainieren;
eine medizinische Vorrichtung den zweiten Datensatz für die Rechenvorrichtung bereitstellt; und
ein Datenspeicher den dritten Datensatz für die Rechenvorrichtung bereitstellt.

5. System nach einem der Ansprüche 1-4, wobei der erste Datensatz ferner wenigstens eines hiervon umfasst: Dicke der Brust, Dichte der Brust oder Patientenpositionierungsinformationen.

6. System nach einem der Ansprüche 1-5, wobei Attribute einer Bildgebungsvorrichtung wenigstens eines hiervon umfassen: Kompressionspaddel-Größe, Kompressionspaddel-Form, Kompressionspaddel-Typ, Bildgebungsmodus, Anodenmaterial oder Bildgebungsfiltertyp.

7. System nach einem der Ansprüche 1-6, wobei der erste Datensatz und der zweite Datensatz während eines aktuellen Patientenbesuchs bei einer Gesundheitseinrichtung gesammelt werden,
und/oder
wobei der dritte Datensatz ferner wenigstens eines von der Dicke der Brust oder der Dichte der Brust umfasst.

8. System nach einem der Ansprüche 1-7, wobei das Maschinenlernmodell dafür trainiert ist, eine optimale Belichtungstechnik zum Erfassen von Mammographiebildern vorherzusagen.

9. System nach einem der Ansprüche 1-8, wobei der Satz von Belichtungstechnikparametern ferner wenigstens eines hiervon umfasst: Brennfleckinformationen, Quelle-zu-Bild-Rezeptorabstandswerte, Objekt-zu-Bild-Rezeptorabstandswerte, Streurasterinformationen oder Strahlbeschränkungsinformationen.

10. Verfahren (400), umfassend:
Empfangen (402), an einer Rechenvorrichtung (105), eines ersten Datensatzes, der patientenspezifische Daten eines Patienten umfasst;
Empfangen (404), an der Rechenvorrichtung (105), eines zweiten Datensatzes, der gerätespezifische Informationen umfasst, wobei die gerätespezifischen Informationen Attribute oder Zubehör einer Bildgebungsvorrichtung angeben;
Empfangen (406), an der Rechenvorrichtung (105), eines dritten Datensatzes, der frühere Bilddaten des Patienten umfasst, wobei die früheren Bilddaten ein oder mehrere Mammographiebilder des Patienten umfassen, wobei die ein oder mehreren Mammographiebilder während eines vorherigen Patientenbesuchs gesammelt wurden;
Bereitstellen (408), durch die Rechenvorrichtung, des ersten Datensatzes, des zweiten Datensatzes und des dritten Datensatzes als Eingabe für ein Maschinenlernmodell;
Empfangen (410) eines Satzes von Belichtungstechnikparametern als Ausgabe von dem Maschinenlernmodell, wobei der Satz von Belichtungstechnikparametern wenigstens eines hiervon umfasst: einen Kilovolt-Spitzenwert (kVp), einen Milliamperesekundenwert (mAs) oder eine Röntgenfilterkombination; und
Generieren (414) eines oder mehrerer Mammographiebilder des Patienten basierend auf dem Satz von Belichtungstechnikparametern, wobei die ein oder mehreren Mammographiebilder durch die Bildgebungsvorrichtung generiert werden.

11. Verfahren nach Anspruch 10, wobei:
die Bildgebungsvorrichtung kommunikativ an die Rechenvorrichtung gekoppelt werden kann; und
die Bildgebungsvorrichtung den zweiten Datensatz für die Rechenvorrichtung bereitstellt, wenn die Bildgebungsvorrichtung und die Rechenvorrichtung kommunikativ gekoppelt sind.

12. Verfahren nach einem der Ansprüche 10-11, wobei die ein oder mehreren Mammographiebilder eine optimale Bildqualität haben, basierend auf wenigstens einem hiervon: optische Dichte, Signal-Rausch-Verhältnis, Kontrast-Rausch-Verhältnis, mittlerer quadratischer Fehler, Kontrastverbesserungsverhältnis.

13. Verfahren nach einem der Ansprüche 10-12, wobei das Verfahren ferner umfasst:
Auswerten des Satzes von Belichtungstechnikparametern unter Verwendung einer Maschinenlernkomponente der Rechenvorrichtung, wobei die Auswertung das Vergleichen des Satzes von Belichtungstechnikparametern mit einem Satz von Parametern für ein traditionelles automatisches Belichtungssteuerungsverfahren (AEC-Verfahren) umfasst.

14. Verfahren nach Anspruch 13, wobei:
das Vergleichen das Kombinieren des Satzes von Belichtungstechnikparametern mit dem Satz von Parametern für das traditionelle AEC-Verfahren umfasst, um einen kombinierten Satz von Belichtungstechnikparametern zu generieren; und
das Verfahren ferner dies umfasst:
Verwenden des kombinierten Satzes von Belichtungstechnikparametern, um einen Satz von Mammographiebildern des Patienten zu generieren.

15. Verfahren nach Anspruch 13, wobei:
das Vergleichen das Verwenden des Satzes von Parametern für das traditionelle AEC-Verfahren umfasst, um statistische Ausreißerwerte in dem Satz von Belichtungstechnikparametern zu identifizieren.

## Revendications

1. Système (100) comprenant :
un processeur ; et
une mémoire couplée au processeur, la mémoire comprenant des instructions exécutables par ordinateur qui, lorsqu'elles sont exécutées, réalisent un procédé comprenant les étapes suivantes :
recevoir (402) un premier ensemble de données comprenant des données spécifiques au patient pour un patient ;
recevoir (404) un deuxième ensemble de données comprenant des informations spécifiques à un équipement, les informations spécifiques à un équipement indiquant des attributs ou des accessoires d'un dispositif d'imagerie ;
recevoir (406) un troisième ensemble de données comprenant des données d'image antérieures du patient, les données d'image antérieures comprenant une ou plusieurs images de mammographie du patient, les une ou plusieurs images de mammographie collectées lors d'une visite précédente du patient ;
fournir (408) le premier ensemble de données, le deuxième ensemble de données, et le troisième ensemble de données en entrée d'un modèle d'apprentissage automatique ;
recevoir (410) un ensemble de paramètres de technique d'exposition en sortie du modèle d'apprentissage automatique, l'ensemble de paramètres de technique d'exposition comprenant au moins l'un des éléments suivants : une valeur de tension de crête (kVp), une valeur de produit courant-temps (mAs) ou une combinaison de filtres à rayons X ; et
acquérir (414) une image du patient sur la base de l'ensemble de paramètres de technique d'exposition.

2. Système selon la revendication 1, dans lequel, avant l'acquisition de l'image, un utilisateur invoque le modèle d'apprentissage automatique.

3. Système selon l'une quelconque des revendications 1 et 2, dans lequel les données spécifiques au patient comprennent un indicateur d'objet étranger, l'indicateur d'objet étranger indiquant si un objet étranger est implanté dans le sein d'un patient.

4. Système selon l'une quelconque des revendications 1 à 3, dans lequel :
un professionnel de santé fournit le premier ensemble de données à un dispositif informatique utilisé pour former le modèle d'apprentissage automatique ;
un dispositif médical fournit le deuxième ensemble de données au dispositif informatique ; et
un magasin de données fournit le troisième ensemble de données au dispositif informatique.

5. Système selon l'une quelconque des revendications 1 à 4, dans lequel le premier ensemble de données comprend en outre au moins une information parmi : l'épaisseur du sein, la densité du sein, ou des informations de positionnement du patient.

6. Système selon l'une quelconque des revendications 1 à 5, dans lequel les attributs d'un dispositif d'imagerie comprennent au moins l'un des attributs suivants : la taille de pale de compression, la forme de pale de compression, le type de pale de compression, le mode d'imagerie, le matériau de l'anode, ou le type de filtre d'imagerie.

7. Système selon l'une quelconque des revendications 1 à 6, dans lequel le premier ensemble de données et le deuxième ensemble de données sont collectés pendant une visite courante d'un patient dans un établissement de soins,
et/ou
dans lequel le troisième ensemble de données comprend en outre au moins l'un parmi l'épaisseur du sein ou la densité du sein.

8. Système selon l'une quelconque des revendications 1 à 7, dans lequel le modèle d'apprentissage automatique est formé pour prédire la technique d'exposition optimale afin d'acquérir des images mammographiques.

9. Système selon l'une quelconque des revendications 1 à 8, dans lequel l'ensemble de paramètres de technique d'exposition comprend en outre au moins l'une des informations suivantes : des informations de point focal, des valeurs de distance source-récepteur d'image, des valeurs de distance objet-récepteur d'image, des informations de grille de diffusion, ou des informations de restriction de faisceau.

10. Procédé (400) comprenant les étapes suivantes :
recevoir (402), au niveau d'un dispositif informatique (105), un premier ensemble de données comprenant des données spécifiques au patient pour un patient ;
recevoir (404), au niveau du dispositif informatique (105), un deuxième ensemble de données comprenant des informations spécifiques à un équipement, les informations spécifiques à un équipement indiquant des attributs ou des accessoires d'un dispositif d'imagerie ;
recevoir (406), au niveau du dispositif informatique (105), un troisième ensemble de données comprenant des données d'image antérieures du patient, les données d'image antérieures comprenant une ou plusieurs images de mammographie du patient, les une ou plusieurs images de mammographie collectées lors d'une visite précédente du patient ;
fournir (408), par le dispositif informatique, le premier ensemble de données, le deuxième ensemble de données, et le troisième ensemble de données en entrée d'un modèle d'apprentissage automatique ;
recevoir (410) un ensemble de paramètres de technique d'exposition en sortie du modèle d'apprentissage automatique, l'ensemble de paramètres de technique d'exposition comprenant au moins l'un des éléments suivants : une valeur de tension de crête (kVp), une valeur de produit courant-temps (mAs) ou une combinaison de filtres à rayons X ; et
générer (414) une ou plusieurs images mammographiques du patient sur la base de l'ensemble de paramètres techniques d'exposition, les une ou plusieurs images mammographiques étant générées par le dispositif d'imagerie.

11. Procédé selon la revendication 10, dans lequel :
le dispositif d'imagerie peut être couplé de manière communicative au dispositif informatique ; et
le dispositif d'imagerie fournit le deuxième ensemble de données au dispositif informatique lorsque le dispositif d'imagerie et le dispositif informatique sont couplés de manière communicative.

12. Procédé selon l'une quelconque des revendications 10 et 11, dans lequel les une ou plusieurs images mammographiques ont une qualité d'image optimale basée sur au moins l'un des éléments suivants : la densité optique, le rapport signal sur bruit, le rapport contraste sur bruit, l'erreur quadratique moyenne, le rapport d'amélioration du contraste.

13. Procédé selon l'une quelconque des revendications 10 à 12, le procédé comprenant en outre les étapes suivantes :
évaluer l'ensemble de paramètres de technique d'exposition au moyen d'un composant d'apprentissage automatique du dispositif informatique, l'évaluation comprenant de comparer l'ensemble de paramètres de technique d'exposition à un ensemble de paramètres pour un procédé classique de commande automatique d'exposition (AEC).

14. Procédé selon la revendication 13, dans lequel :
la comparaison comprend de combiner l'ensemble de paramètres de technique d'exposition avec l'ensemble de paramètres pour le procédé classique AEC afin de générer un ensemble combiné de paramètres de technique d'exposition ; et
le procédé comprenant en outre l'étape suivante :
utiliser l'ensemble combiné de paramètres de technique d'exposition pour générer un ensemble d'images mammographiques du patient.

15. Procédé selon la revendication 13, dans lequel :
la comparaison comprend d'utiliser l'ensemble de paramètres pour le procédé AEC classique afin d'identifier des valeurs statistiquement aberrantes dans l'ensemble des paramètres de technique d'exposition.
